# EUROPEAN PATENT APPLICATION

(11) **EP 3 836 156 A1**
(43) Date of publication of application: **16.06.2021**
(21) Application number: 19383091.6
(22) Date of filing: 10.12.2019
(51) Int. Cl.: G16H 40/20, G16H 40/40

(54) **SYSTEMS AND METHODS FOR PROVIDING TRANSPARENT MEDICAL TREATMENT**

(71) Applicant: F. Hoffmann-La Roche AG, 4070 Basel (CH); Roche Diagnostics GmbH, 68305 Mannheim (DE)
(72) Inventor: BLANCO, Fernando, 08174 San Cugat del Valles Barcelona (ES)
(74) Representative: Herren, Barbara

(57) **Abstract**

A method for tracking availability of a point-of-care (POC) device for a user is provided. The method comprises entering type(s) of test(s) needed by the user, providing a list of available POC devices to the user based on the type(s) of test(s) needed, based on the list of available POC devices, providing a map to the user showing locations of nearby POC devices with availability for the type(s) of test(s) needed; selecting one of the nearby POC devices by touching the location of the POC device on the map and booking that POC device for the type(s) of test(s) needed at a particular time slot, and communicating the booking to the selected POC device. The selected POC device then blocks that time slot.

## Description

### Technical Field

The present disclosure generally relates to providing point-of-care (POC) medical treatment to patients and, in particular, to providing localize point-of-care medical treatment to patients based on the location of the patients and a POC facility.

### Background

In vitro diagnostic testing has a major effect on clinical decisions by providing physicians with pivotal information. Particularly, there is great emphasis on providing quick and accurate test results in critical care settings.

One particular type of diagnostic testing is bedside testing or point of care (POC) testing. This type of diagnostic testing is performed mainly by nurses or medical staff primarily trained to operate the instruments available at the site of patient care, such as hospitals, emergency departments, intensive care units, primary care setting, medical centers, patient homes, a physician's office, a pharmacy or a site of an emergency. Major benefits are obtained when the measurement results obtained by a POC testing device(s) are made available immediately/results can be shared instantaneously with all members of the medical team, thereby enhancing communication through decreasing turnaround time.

POC testing has become established worldwide and finds vital roles in public health. Potential operational benefits of POC testing include: faster decision making, reduced operating times, reduced postoperative care time, reduced emergency room time, reduced number of outpatient clinic visits, reduced number of hospital beds required and overall optimal use of professional time.

Generally, the goal of Point of Care is to help both healthcare professionals and patients achieve improved clinical and health-economic outcomes, by delivering robust, connected, easy to use point of care solutions outside the central lab, providing immediate results and thus allowing treatment decisions to be made more quickly - inside or outside the hospital. Point of Care testing delivers those solutions meeting the clinical need for quick and accurate test results delivered where needed, when needed; on the device, in an electronic healthcare record on a patient/ward monitor, to the clinician on the move and directly to the patient.

Some patients with chronic illnesses or medical conditions require ongoing POC testing for disease maintenance and management. These tests can be ordered by their medical professionals overseeing the patients' care. Such testing typically can take place at point of care (POC) facilities that the patients may have to travel to have their testing done. The POC facilities may have a variety of different POC devices available to conduct testing. The different POC devices can conduct different clinical tests. The different clinical tests require different reagents and quality controls (QC) to ensure proper testing. The patients may travel to a POC facility only to discover that POC facility does not have the correct POC testing device to conduct the test needed by the patient or that the POC device is not configured or available to run the needed test at the time the patient arrives at the POC facility due to lack of reagents or inadequate QC on that POC device.

Unfortunately, currently, there is nothing available that can provide an integrated view of the POC devices available to users to conduct the necessary testing at a specific POC facility within the current vicinity of the patient. This situation can create issues regarding the efficiency of patient care since the patient cannot have access to fast test results since s/he is not aware of which POC facilities have the required POC devices to conduct the needed patient testing within the vicinity of the patient resulting in unnecessary trips to different POC facilities in search of a POC facility with available POC devices.

In addition, because POC devices are normally isolated, or have low connectivity to the outside world, the user does not have the means to know where the required POC devices are located and/or whether the POC devices are available for testing. There is currently no solution to see all of the required and available POC devices in a certain area/vicinity forming the patient's common ecosystem.

Therefore, there is a need for a POC device location system that allows a patient to find the nearest POC facility with a required and available POC device in order to have the required testing needed done.

### Sum mary

According to the present disclosure, a method for tracking availability of a point-of-care (POC) device for a user is presented. The method can comprise the steps of entering type(s) of test(s) needed by the user, determining a geolocation of the patient, providing a list of POC facilities with available POC devices to the user based on the type(s) of test(s) needed and the geolocation of the patient, based on the list of POC facilities with available POC devices, providing a map to the user showing locations of nearby POC devices with availability for the type(s) of test(s) needed near the geolocation of the patient, selecting one of the nearby POC devices by touching the location of the POC device on the map and booking that POC device for the type(s) of test(s) needed at a particular time slot, and communicating the booking to the selected POC device, wherein the selected POC device blocks that time slot.

The method can further comprise the step of connecting several cloud services of various POC systems to track the personal health record of a patient.

The method can further comprise the step of providing an information channel in which a POC device sends relevant information regarding patients and usage.

The method can further comprise the addition step of, after the type(s) of test(s) needed by the user is performed, rating the method of selection of POC facility by the user as well as providing a means for the patient to review the POC facility experience, wherein the user can be a patient. The list of POC facilities with available POC devices can be driven by geolocation position of the patient, the type(s) of test(s) needed, medical urgency of the type(s) of test(s), geolocation position of the POC devices, health insurance coverage of the patient; requested date and/or time of the test(s); price range, acceptable time margin that the patient is willing to accept, or combinations thereof.

The method can further comprise receiving test results from the POC device, after the test(s) needed by the user is performed, regarding the patient's personal health and receiving notifications regarding current trends of the patient's personal health to provide advice for future testing.

The method can further comprise the additional step of, after the type(s) of test(s) needed by the user is performed, rating the method of selection of POC facility by the user as well as providing a means for the patient to review the POC facility experience, wherein the user is a medical professional. The list of POC facilities with available POC devices can be driven by geolocation position of the medical professional and/or a patient's preferred location, the type(s) of test(s) needed by the user, medical urgency of the type(s) of test(s), type of POC device needed, geolocation position of the POC devices, health insurance coverage of the patient; requested date and/or time of the test(s), price range, acceptable time margin for an appointment, or combinations thereof.

The method can further comprise monitoring the test results from the POC device, after the test(s) needed is performed, regarding personal health of tested patient and notifying the patient with test updates and/or rescheduling options.

The POC device can be physically resident in a point of care (POC) facility. The POC facility can comprise a plurality of POC devices, a plurality of reagents, and a connection to a cloud to communicate with the user. The POC facility can be a small medical clinic and/or a pharmacy

The method can further comprise managing requirements of the plurality of POC devices and the plurality of reagents for the POC facility and notifying when there are shortages of POC devices and/or reagents.

The method can further comprise extracting information regarding instrument behavior regarding accuracy of the POC devices and/or quality control of the POC devices, generating recommendations regarding other type(s) of test(s) to optimize the test(s) and/or other possible suitable POC devices and/or other possible type(s) of quality control based on the extracted instrument behavior information; and sending the instrument behavior information and recommendations to the POC device manufacturer.

The method can further comprises extracting information from external data sources such as, for example, from the Internet, to obtain public data such as, for example, current weather conditions, diseases such as current local flu and cold activity, current local allergens/pollen levels, and other types of public data that might have an influence on the usage of the POC system and/or particular testing needs.

The method can further comprise pushing messages to the user comprising updates on possible tests for the patient that may be available and/or rescheduling options and/or commercial updates.

The method can further comprise notifying the POC facility about the need to add new device(s) to the POC facility based on current trends and the historical data of that POC facility in order to satisfy future demands of a certain type(s) of test(s).

The method can further comprise suggesting to the patient that s/he may need to schedule the next planned test of a first test at another different POC facility which has also a POC device capable and available of making a secondary test such as, for example, rerun/reflex testing, if the test result of a first type reaches a high/low measurement limit of the test performed at the current POC facility.

### Brief Description of the Several Views of the Drawings

The following detailed description of specific embodiments of the present disclosure can be best understood when read in conjunction with the following drawings, where like structure is indicated with like reference numerals and in which:
Fig. 1 illustrates a general overview of the POC system according to an embodiment of the present disclosure.
Fig. 2 illustrates a flowchart of the method of selecting an available POC facility according to an embodiment of the present disclosure.
Figs. 3A-H illustrate a series of GUI screen images for a typical application of the method of finding an available nearby POC facility according to an embodiment of the present disclosure.

### Detailed Description

In the following detailed description of the embodiments, reference is made to the accompanying drawings that form a part hereof, and in which are shown by way of illustration, and not by way of limitation, specific embodiments in which the disclosure may be practiced. It is to be understood that other embodiments may be utilized and that logical, mechanical and electrical changes may be made without departing from the spirit and scope of the present disclosure.

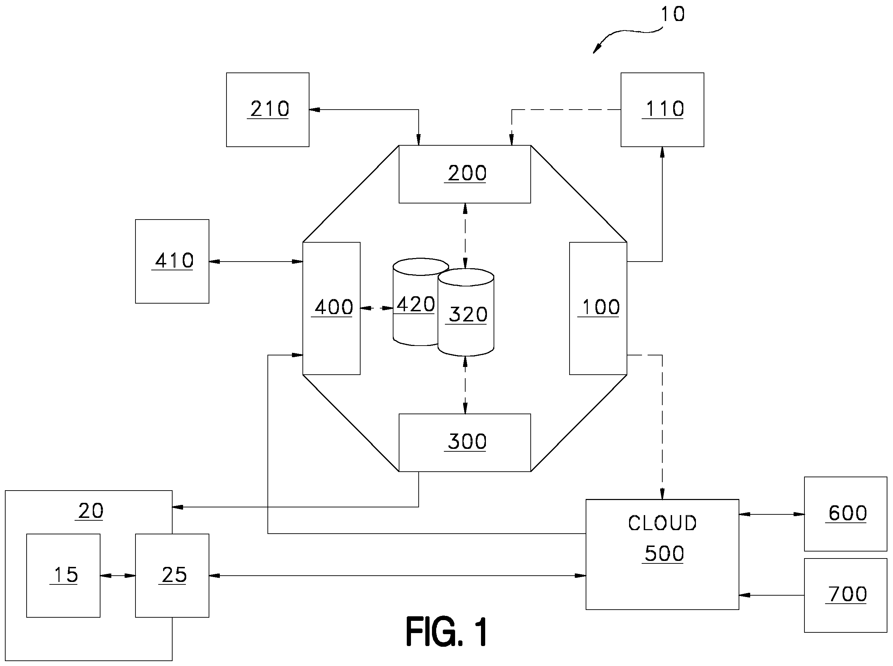

Referring initially to Fig. 1, Fig. 1 illustrates a general overview of a Point of Care (POC) system 10. The POC system 10 can provide several different types of services such as, for example, patient services 100, medical professional services 200, POC station services 300, and insight services 400.

The term 'point of care (POC)' or 'point of care (POC) environment' as used herein can be defined to mean a location on or near a site of patient care where medical or medically related services such as medical testing and/or treatment can be provided, including but not limited to hospitals, emergency departments, intensive care units, primary care setting, medical centers, patient homes, a physician's office, a pharmacy or a site of an emergency.

The term 'point of care testing (POCT)' as used herein can encompass analysis of one or more patient(s) or patient sample(s) for one or more patient health parameters in a point of care (POC) environment. Point of care testing (POCT) can be often accomplished through the use of transportable, portable, and handheld analytical devices, but small bench-top analyzers or fixed equipment can also be used when a handheld device is not available- the goal being to collect the patient health parameter and obtain analytical data in a (relatively) short period of time at or (relatively) near the location of the patient.

The terms 'sample', 'patient sample' and 'biological sample' can refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

The term 'analysis' or 'analytical test' as used herein can encompass a laboratory procedure characterizing a parameter of a biological sample, e.g., light absorption, fluorescence, electrical potential or other physical or chemical characteristics of the reaction to provide the measurement data.

The term 'analytical device' as used herein can refer to an apparatus configured to obtain a measurement value. An analytical device can be operable to determine via various chemical, biological, physical, optical, electro-chemical or other technical procedures a parameter value of the sample or a component thereof. An analytical device may be operable to measure the parameter of the sample or of at least one analyte and return the obtained measurement value. The list of possible analysis results returned by the analytical device can comprise, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical device may comprise units assisting with the pipetting, dosing, and mixing of samples and/or reagents. The analytical device may comprise a reagent holding unit for holding reagents to perform the assays.

A Point of Care (POC) facility 20 can be a location at which several Point of Care (POC) devices 15 can be physically located. As described above, a POC devices 15 can be any transportable, portable, and handheld diagnostic devices used to measure and obtain diagnostic results quickly and typically in the presence of the patient so that the patient can receives fast feedback from a diagnostic test. Examples of POC devices 15 can be a blood glucose meter, a nerve conduction device, a blood coagulation meter, and a test kit such as for rapid strep test, pregnancy tests, cardiac biomarker tests, and the like.

The POC facility 20 can exist in several different environments such as, for example, pharmacies and/or small health care facilities such as, for example, doctor's offices, urgent care clinics, workplace wellness centers, and the like. The POC facility 20 can also be a residence of a self-testing patient with his/her own POC devices 15.

The POC facility 20 can also include a means to communicate 25 with the cloud 500 such as, for example, a communication network, for example, by cobas IT-1000™, in order to convey, for example, POC device 15 data, test data, quality control (QC) data, and the like. The cloud 500, in turn, can communicate the conveyed data to the overall POC system 10 for storage in POC station database 320. The term communication network' as used herein can encompass any type of wireless network, such as a WIFI, GSM, UMTS or a cable based network, such as Ethernet or the like. In particular, the communication network can implement the Internet protocol IP. For example, the communication network can comprise a combination of cable-based and wireless networks. In embodiments wherein units of the system are comprised within one laboratory instrument, the communication network can comprise communication channels within an instrument.

The POC facility 20 can have a stock of reagents typically used by the POC devices 15. The POC facility 20 can communicate via the cloud 500 with the POC station services 300 to update the usage of the POC devices 15 and the reagents housed at that particular POC facility 20 in the POC facility database 320. In addition, the POC facility 20 can communication with a POC facility coordinator 700 via the cloud 500 about testing and usage trends and alert the POC facility coordinator 700 with predictions about low/empty stocks of reagents and other consumables, predictions about increases of the test demand and/or reagent usage, and the like. The POC facility coordinator 700 can then act on the information supplied by the POC facilities 20.

Each POC facility 20 can have its own internal calendar to maintain and schedule the available tests provided by the POC devices 15 resident at that particular POC facility 20. Further, the POC facility 20 can receive information about all the POC facility agendas from the POC station services 300.

Each POC facility 20 can have a group of patients 110 associated with it for which that POC facility 20 can be the closest testing facility for that particular group of patients 110.

Each of the POC facilities 20 in a plurality of POC facilities 20 can be in a hierarchy with each other.

The POC data created from each POC facility 20 can be submitted into a cloud 500 by the means of communication 25. The relevant created POC data can then be used to further expand the insight services 400 in the POC system 10 such as, for example, usage patterns regarding tests by patient type and location or reagent usage trends. The relevant created POC data can also be used to generate recommendations regarding other type(s) of test(s) to optimize the test(s) and/or other possible suitable POC devices and/or other possible type(s) of quality control based on the extracted instrument behavior information These insights services 400 can then be sent to the manufacturer 410 of the POC system 10 in order to create detailed business intelligence reports for the manufacturer 410.

The POC system 10 can also provide patient services 100 to the group of patients 110. The patient services 100 can allow each individual patient in the group of patients 110 the ability to book the test(s) needed by the patient. In addition, the patient services 100 can allow each individual patient in the group of patients 110 the ability to search for the closest POC facility 20 or the POC facility 20 that offers the best match to that individual patient's needs. Such needs can be, for example, available times at the POC facility 20, costs, and/or types of health insurance accepted by that POC facility 20. Further, the patient services 100 can provide a means for the group of patients 110 to get information regarding their own personal health records stored on the POC system 10.

The POC system 10 can also provide medical professional services 200 to the group of medical professionals 210. The medical professionals can be, for example, physicians, nurses, nurse practitioners, physician assistants, or any other medical professionals who may be involved with the health care management of a patient. The medical professional services 200 can allow a medical professional 210 to request that a patient test be conducted on a patient and can allow for that the medical professional 210 to book that patient test for the patient at a POC facility 20 that has an available POC device 15. In addition, the medical professional services 200 can allow a medical professional 210 to monitor the personal health records of a patient. Further, medical professional services 200 can allow a medical professional 210 to manage the agenda of the medical professional 210 him/herself.

The POC system 10 will be able to interconnect between the different services with a means of communication management such as, for example, IT-1000™, in order to import data from the different services offered by the POC system 10 that can be stored, for example, in databases such as an insight database 420 or the POC facility database 320, as well as with the different POC instruments 15 located at the POC facilities 20 and the POC device 15 manufacturer 410.

The POC system 10 can also connect with several external data sources that are currently commonly available over the Internet in order to obtain public data such as, for example, current weather conditions, diseases such as current local flu and cold activity, current local allergens/pollen levels, and other types of public data that might have an influence on the usage of POC devices 15.

The POC system 10 can provide its services via apps that can provide patients and medical professionals with a set of software services to manage their own results, find the nearest POC facilities, and share their medical information. The apps can be found on the patient's and/or medical professional's personal computing devices such as, for example, mobile phones, tablets, and/or personal computers.

In one embodiment, two different algorithms can be created: a patient search engine and a medical professional search engine. Both search engines can return a set of data in order to fulfill the graphical component application programming interface (API) requirement of the app in order to draw the information on the GUI screen of the personal computing device so that the patient and/or medical professional can select an available POC device at a POC facility easily and efficiently.

In one embodiment, as the POC system can be designed following the Command Query Responsibility Segregation (CQRS) pattern, there can be two different POC ecosystem storage systems, one for each engine in order to simplify the development and maintenance for each engine.

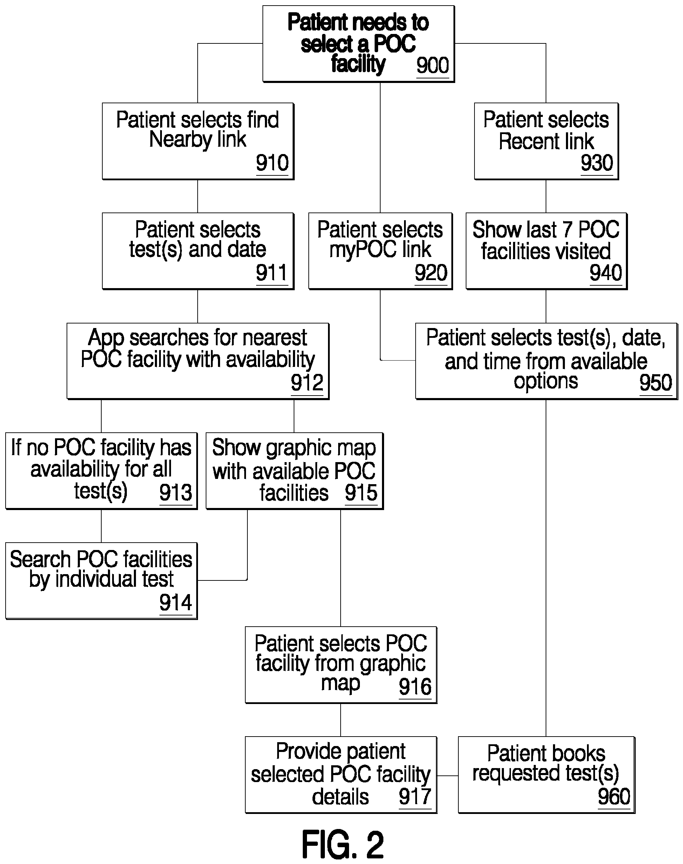

With the patient search engine, the patient can be able to search the POC system for a POC facility having a POC device with the capability and availability to perform the test required by the patient. Fig. 2 illustrates the workflow for that can allow a patient to select a POC facility with an available POC device. Starting at the homepage of the app on the patient's personal computing device (and as shown in Fig. 2B), at step 900, the patient may select one of three options to select a POC facility: Nearby, MyPOC, or Recent, using a user interface. The term 'user interface' as used herein can encompass any suitable piece of software and/or hardware for interactions between an operator and a machine, including but not limited to a graphical user interface for receiving as input a command from an operator and also to provide feedback and convey information thereto. Also, a system device may expose several user interfaces to serve different kinds of users/operators.

If the patient selects the Nearby option in step 910, initially, the patient can enter the type(s) of test(s) that the patient needs to have performed as well as, for example, the date the patient would prefer to have the test(s) performed in step 911. The API system can the provide search results to the patient in step 912. The criteria driving the API searches can include, for example, patient position (i.e., the current geolocation of the patient), the type(s) of required test(s), POC facility position (i.e., the geolocation of the POC facility), the insurance coverage of the patient if any, the requested date/time of the required test(s), the price range of the required test(s), the acceptable time margin in which the patient is willing to accept to book a test, the medical urgency of the type(s) of test(s) such as, for example, a type of test that needs to performed at a certain frequency, such as, for example, every day or every three days, and/or combinations thereof.

Based on the above criteria, the app can provide the patient with a graphic map showing icons of the physical locations of any POC facilities having available POC devices matching the search requirements for the required test(s) in step 915. The patient can then select a POC facility by tapping one of the POC facilities graphically displayed on the map in step 916. Once the patient selects a POC facility from the map, the patient can be provided with details of the selected POC facility in step 917 such as, for example, hours, address, costs of the test(s), and the like. The patient can then proceed with booking the test(s) at the selected POC facility at step 960.

Once the patient selects a POC facility, the patient can then book the test(s) at that POC facility. The booking of the test(s) can then be communicated to the POC facility that supports the available POC device(s) that can provide the required test(s) via the app through a cloud solution, for example. The selected POC facility can then block/reserve the available POC device(s) needed to perform the required test(s) for the patient during the requested time period from other use.

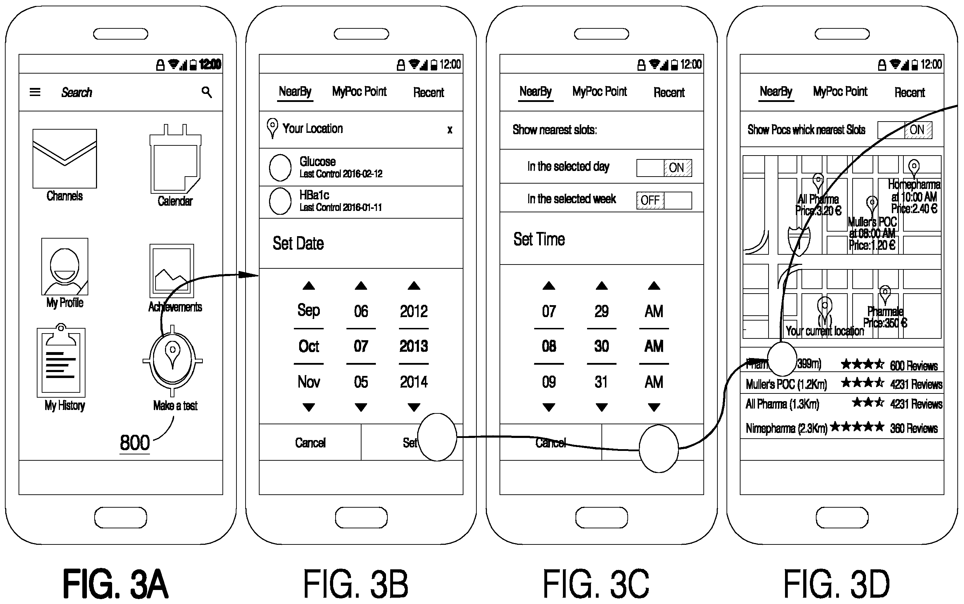

Turning to Figs. 3A-H, Figs. 3A-H illustrate an exemplary series of GUIs shown on the patient's personal computing device when the patient wants to find a nearby POC facility. Fig. 3A illustrates what a typical home screen of the patient may resemble with the Make a Test app icon 800. Initially, the patient, in Fig. 3A, can select the Make a Test icon 800 on his/her home screen. At the next screen, illustrated in Fig. 3B, the patient can search for POC facilities nearby by selecting the Nearby link on GUI page illustrated in Fig. 3B. The patient can enter the type(s) of test(s) the patient needs to have performed on the POC device, for example, Glucose and HBalc, and the date on which the patient would like the test(s) performed. On the next screen, as illustrated on Fig. 3C, the patient can then select the time at which he/she would like to have the test(s) performed on the selected date.

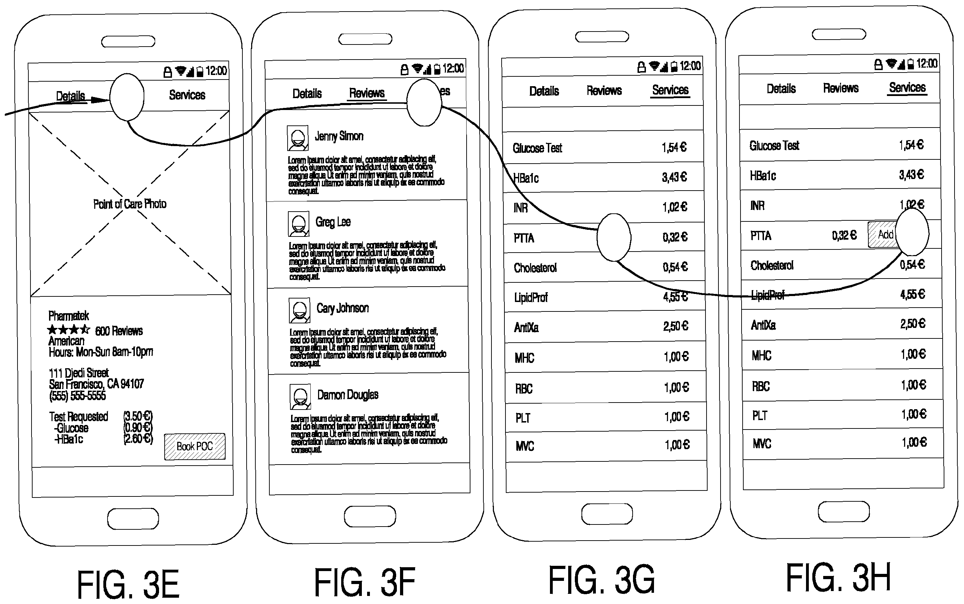

The API system can then provide nearby POC facilities with available POC devices results to the patient that match the requested criteria of test(s), date, and time as shown in Fig. 3D. Based on the above criteria, the app can provide the patient with a graphic map showing icons of the physical locations of any nearby POC facilities matching the search requirements for the required test(s) as illustrated in Fig. 3D. The patient can then select a POC facility by tapping on one of the POC facilities icons graphically displayed on the map. Detailed information regarding the selected POC facility will then be displayed on the patient's personal computing device as illustrated in Fig. 3E. The patient may also read reviews of the selected POC facility, as illustrated in Fig. 3F, by selecting the reviews link provided on Fig. 3E. In addition, the patient may also view all the services provided by the selected POC facility and the costs associated with the provided services by selecting the services link on either the POC facility details page (Fig. 3E) or the POC facility reviews page (Fig 3F). An exemplary POC facility services page is illustrated in Fig. 3G. On the exemplary POC facility services page, the patient may select an additional test such as, for example, PTTA. The patient will then be shown a confirmation page, as illustrated in Fig. 3H, confirming whether the patient does indeed want to add that additional test. Following the confirmation of the test(s) the patient wants performed, the test(s) can then be booked at the POC facility.

Referring back to Fig. 2, if the patient selects the Recent option at step 930 (and as shown in Fig. 3B) instead of the Nearby link, the last seven POC facilities visited by the patient can be listed on the screen of the patient's personal computing device in step 940 either graphically or as a list. The patient can then select one of these seven POC facilities and can be presented with the available test(s), dates, and times at that selected POC facility at step 950. The patient can then select the desired test(s), dates, and times at step 950 at that POC facility and book that requested test(s) at step 960.

Again referring to Fig. 2, if the patient chooses the MyPOC option at step 920 (and as shown in Fig. 3B) instead of the Nearby or Recent link, the patient's preferred POC facility can already be selected and displayed. This option can eliminate the steps in which the patient needs to select a POC facility as discussed above. With myPOC, the patient can then be shown the available test(s), dates, and times at that pre-selected POC facility. The patient can select the desired test(s), dates, and times at step 950 at that POC facility and book that requested test(s) at step 960.

In one embodiment, after all the required test(s) have been conducted, the patient can then rate the testing experience in the POC system via the app as well as provide a review of the POC facility.

With the medical professional search engine, the medical professional can book one or more tests for one of medical professional's patients a POC facility having available POC devices. The method can be similar to the method needed for the patient search engine as discussed above. However, the POC system can provide search results to the medical professional, instead of the patient. The criteria driving the API searches for the medical professional search engine can be, for example, the position of the medical professional (i.e., the geolocation of the medical professional) or a patient preferred location, the type(s) of required test(s), medical urgency of the type(s) of test(s), the type(s) of POC device(s) located at the POC facility, the precision of the POC device(s) located the POC facility, POC facility position (i.e., the geolocation of the POC facility, the price range of the required test(s), the insurance coverage of the patient if any, the requested date of the required test(s), an acceptable time margin for the appointment to occur, and/or combinations thereof.

In one embodiment, the POC system can be capable of sending push messages to the personal computing systems of some users such as, for example, medical professionals, with updates on possible new tests available and/or rescheduling options and/or commercial updates. In another embodiment, the messaging system may be visible to patients as well. The message system may also send updates and reminders to the patient.

In another embodiment, the POC system can be capable of notifying the POC facility about the need to add new device(s) to the POC facility based on current trends and the historical data of that POC facility in order to satisfy future demands of a certain type(s) of test(s). For example, if last summer, a POC facility was close to reaching the limit of tests that could be made with POC facility's Accuchek™ and this year it is predicted that there will be an increase of 10% for Accuchek™ tests, the POC system can notify the POC facility that additional Accuchek™ devices may be needed.

In another embodiment, if a test result of a first type reaches a high/low measurement limit of the test performed, it can be mandatory to repeat the test or to order a secondary test. In this situation, the POC system can suggest to the patient to schedule the next planned test of the first test at another POC facility which has also a POC device capable and available of making the secondary test such as, for example, rerun/reflex testing.

For example, if based on the trend of the glucose test results of a patient that is highly probable the next test will reach the limit of that test and at the patient's preferred POC facility no POC device capable to make the secondary test is available but at a nearby POC facility there is such a POC device, the POC system can suggest to the patient to use this nearby POC facility to conduct the next glucose test along with any necessary secondary testing.

Further disclosed and proposed is a computer program including computer-executable instructions for performing the method according to the present disclosure in one or more of the embodiments enclosed herein when the program is executed on a computer or computer network. Specifically, the computer program may be stored on a computer-readable data carrier. Thus, specifically, one, more than one or even all of method steps as disclosed herein may be performed by using a computer or a computer network, preferably by using a computer program.

Further disclosed and proposed is a computer program product having program code, in order to perform the method according to the present disclosure in one or more of the embodiments enclosed herein when the program is executed on a computer or computer network. Specifically, the program code may be stored on a computer-readable data carrier.

Further disclosed and proposed is a data carrier having a data structure stored thereon, which, after loading into a computer or computer network, such as into a working memory or main memory of the computer or computer network, may execute the method according to one or more of the embodiments disclosed herein.

Further disclosed and proposed is a computer program product with program code stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Further disclosed and proposed is a modulated data signal which contains instructions readable by a computer system or computer network, for performing the method according to one or more of the embodiments disclosed herein.

Referring to the computer-implemented aspects of the present disclosure, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing measurements.

Further disclosed and proposed is a computer or computer network comprising at least one processor, wherein the processor is adapted to perform the method according to one of the embodiments described in this description.

Further disclosed and proposed is a computer loadable data structure that is adapted to perform the method according to one of the embodiments described in this description while the data structure is being executed on a computer.

Further disclosed and proposed is a storage medium, wherein a data structure is stored on the storage medium and wherein the data structure is adapted to perform the method according to one of the embodiments described in this description after having been loaded into a main and/or working storage of a computer or of a computer network.

It is noted that terms like "preferably," "commonly," and "typically" are not utilized herein to limit the scope of the claimed embodiments or to imply that certain features are critical, essential, or even important to the structure or function of the claimed embodiments. Rather, these terms are merely intended to highlight alternative or additional features that may or may not be utilized in a particular embodiment of the present disclosure.

Having described the present disclosure in detail and by reference to specific embodiments thereof, it will be apparent that modifications and variations are possible without departing from the scope of the disclosure defined in the appended claims. More specifically, although some aspects of the present disclosure are identified herein as preferred or particularly advantageous, it is contemplated that the present disclosure is not necessarily limited to these preferred aspects of the disclosure.

## Claims

1. A method for tracking availability of a point-of-care (POC) device for a user, the method comprising:
entering type(s) of test(s) needed by a patient;
determining a geolocation position of the patient;
providing a list of POC facilities with available POC devices to the user based on the type(s) of test(s) needed and the geolocation position of the patient;
based on the list of POC facilities with available POC devices, providing a map to the user showing physical locations of POC facilities with POC devices with availability for the type(s) of test(s) needed near the geolocation position of the patient;
selecting one of the nearby POC devices from the list of POC facilities with available POC devices by touching the map location of the POC facility on the map and booking that POC device for the type(s) of test(s) needed at a particular time slot at that POC facility; and
communicating the booking of the selected POC device to POC facility, wherein the selected POC device blocks that time slot from other users to select.

2. The method according to claim 1, further comprising,
providing an information channel in which a POC device sends relevant information regarding patients and usage to a POC storage database.

3. The method according to claim 1, further comprising,
after the type(s) of test(s) needed is/are performed, rating testing experience at the selected POC facility by the user as well as provide a review of the POC facility by the user.

4. The method according to claim 1, wherein the user is the patient.

5. The method according to claim 4, wherein the list of POC facilities with available POC devices is driven by geolocation position of the patient, the type(s) of test(s) needed, medical urgency of the type(s) of test(s), geolocation position of the POC devices, insurance of the patient; requested date and/or time; price range, acceptable time margin that the patient is willing to accept, or combinations thereof.

6. The method according to claims 4 and 5, further comprising,
receiving test results from the POC device, after the test(s) needed is performed, regarding the patient's personal health; and
receiving notifications regarding current trends of the patient's personal health to provide advice for future testing.

7. The method according to claim 1, wherein the user is a medical professional.

8. The method according to claim 7, wherein the list of POC facilities with available POC devices is driven by geolocation position of the medical professional and/or a patient's preferred location, the type(s) of test(s) needed, type of POC device; geolocation position of the POC devices, insurance of the patient; requested date and/or time; price range, acceptable time margin for an appointment, medical urgency of the type(s) of test(s); or combinations thereof.

9. The method according to claims 7 and 8, further comprising,
monitoring the test results from the POC device, after the test(s) needed is performed, regarding personal health of tested patient; and
notifying the patient with test updates and/or rescheduling options.

10. The method of claim 1, further comprising,
extracting information from external data sources to obtain public data.

11. The method according to claim 1, wherein the POC device is physically resident in a point of care (POC) facility.

12. The method according to claim 1, wherein the POC facility comprises a plurality of POC devices, a plurality of reagents, and a connection to a cloud to communicate with the user.

13. The method according to claim 12, further comprising,
managing requirements of the plurality of POC devices and the plurality of reagents for the POC facility; and
notifying when there are shortages of POC devices and/or reagents.

14. The method according to claims 12 and 13, further comprising,
extracting information regarding instrument behavior;
generating recommendations regarding other type(s) of test(s) and/or other POC devices and/or other type(s) of quality control based on the extracted instrument behavior information; and
sending the instrument behavior information and recommendations to POC device manufacturer.

15. The method according to claim 12, wherein the POC facility is a small medical clinic or a pharmacy.
